# EUROPEAN PATENT SPECIFICATION

(11) **EP 2 443 251 B1**
(45) Date of publication and mention of the grant of the patent: **13.08.2014**
(21) Application number: 10723147.4
(22) Date of filing: 15.06.2010
(51) Int. Cl.: C12Q 1/68, C12N 15/10

(54) **IMPROVEMENT OF LOW-BIOMASS SOIL DNA/RNA EXTRACTION YIELD AND QUALITY**
VERBESSERUNG DER AUSBEUTE UND QUALITÄT DER DNA/RNA-EXTRAKTION AUS ERDE MIT GERINGER BIOMASSE
AMÉLIORATION DE LA QUALITÉ ET DU RENDEMENT D'EXTRACTION ADN/ARN DE SOL À FAIBLE TENEUR EN BIOMASSE

(30) Priority: 02.07.2009 US 222649 P; 15.06.2009 EP 09162686; 16.03.2010 EP 10156628
(43) Date of publication of application: 25.04.2012
(73) Proprietor: De Nationale Geologiske Undersøgelser For Danmark Og Grønland, 1350 Copenhagen K (DK)
(72) Inventor: BÆLUM, Jacob, DK-1671 Copenhagen V (DK); JACOBSEN, Carsten Suhr, DK-2700 Brønshøj (DK)
(74) Representative: Inspicos A/S
(86) International application number: PCT/EP2010/058336
(87) International publication number: WO 2010/146026

(56) References cited:
- US-A- 5 753 466
- HOSHINO ET AL: "DNA- versus RNA-based denaturing gradient gel electrophoresis profiles of a bacterial community during replenishment after soil fumigation" SOIL BIOLOGY AND BIOCHEMISTRY, PERGAMON, OXFORD, GB, vol. 39, no. 2, 29 November 2006 (2006-11-29), pages 434-444, XP005783596 ISSN: 0038-0717 cited in the application
- ROCKLAND IMMUNOCHEMICALS FOR RESEARCH: "Salmon sperm DNA ready-to-use solution" 2008, XP002546414 Retrieved from the Internet: URL:www.rockland-inc.com/objects/catalog/p roduct/extras/12259_MB-103-0025.pdf [retrieved on 2009-09-18]
- GE Healthcare: "Hybridization Reagents" 2008, XP002592972 Retrieved from the Internet: URL:http://www.gelifesciences.com/aptrix/u pp01077.nsf/Content/na_blotting_site~na_hy bridization~na_reagents_hybridization [retrieved on 2010-07-20]
- GOLENBERG E M ET AL: "Effect of highly fragmented DNA on PCR." NUCLEIC ACIDS RESEARCH, vol. 24, no. 24, 15 December 1996 (1996-12-15), pages 5026-5033, XP001525319 ISSN: 0305-1048
- FROST M R ET AL: "Prevention of depurination during elution facilitates the reamplification of DNA from differential display gels." NUCLEIC ACIDS RESEARCH, vol. 27, no. 15, 1 August 1999 (1999-08-01), page E6, XP002592973 ISSN: 1362-4962
- RYOJI M ET AL: "Repair of DNA damage in a mitochondrial lysate of Xenopus laevis oocytes." NUCLEIC ACIDS RESEARCH, vol. 24, no. 20, 15 October 1996 (1996-10-15), pages 4057-4062, XP001525318 ISSN: 0305-1048
- BARTON H A ET AL: "DNA extraction from low-biomass carbonate rock: An improved method with reduced contamination and the low-biomass contaminant database" JOURNAL OF MICROBIOLOGICAL METHODS, ELSEVIER, AMSTERDAM, NL LNKD- DOI:10.1016/J.MIMET.2005.10.005, vol. 66, no. 1, 1 July 2006 (2006-07-01), pages 21-31, XP025073202 ISSN: 0167-7012 [retrieved on 2006-07-01]
- NANASSY ET AL: "Capture of genomic DNA on glass microscope slides" ANALYTICAL BIOCHEMISTRY, ACADEMIC PRESS INC, NEW YORK, vol. 365, no. 2, 10 May 2007 (2007-05-10), pages 240-245, XP022077173 ISSN: 0003-2697
- DIZDAROGLU M ET AL: "Separation and sequencing of the sequence isomers of pyrimidine deoxypentanucleoside tetraphosphates by high-performance liquid chromatography", JOURNAL OF CHROMATOGRAPHY, ELSEVIER SCIENCE PUBLISHERS B.V, NL, vol. 188, no. 1, 25 January 1980 (1980-01-25), pages 273-279, XP026550079, ISSN: 0021-9673, DOI: 10.1016/S0021-9673(00)88442-3 [retrieved on 1980-01-25]

### SEQUENCE LISTING

<110> De nationale geologiske undersogelser for Danmark og Grenland
<120> IMPROVEMENT OF LOW-BIOMASS SOIL DNA/RNA EXTRACTION YIELD AND QUALITY
<130> 17929PCT00
<160> 5
<170> PatentIn version 3.5
<210> 1
   <211> 18
   <212> DNA
   <213> Artificial sequence
<220>
   <223> Synthetic PCR primer
<400> 1
   gggagtatcg accctctc 18
<210> 2
   <211> 21
   <212> DNA
   <213> Artificial sequence
<220>
   <223> Synthetic PCR primer
<400> 2
   ggattagctc cagttcacac t 21
<210> 3
   <211> 18
   <212> DNA
   <213> Artificial sequence
<220>
   <223> Synthetic PCR primer
<400> 3
   ggattagctc cagttcac 18
<210> 4
   <211> 15
   <212> DNA
   <213> Artificial sequence
<220>
   <223> Synthetic PCR primer
<400> 4
   cctacgggag gcagc 15
<210> 5
   <211> 15
   <212> DNA
   <213> Artificial sequence
<220>
   <223> Synthetic PCR primer
<400> 5
   attaccgcgg ctgct 15

## Description

### FIELD OF THE INVENTION

The present invention relates to the field of extraction of DNA/RNA from samples, in particular highly adsorptive samples like low-biomass soil samples. The present invention provides a pre-treatment step which - in a cost-efficient manner - renders it possible to improve the yield and quality of the DNA/RNA extraction.

### BACKGROUND OF THE INVENTION

Extraction of DNA/RNA from low-biomass samples, e.g. from clay-rich subsoil samples, poses various problems, i.a. that DNA/RNA tends to become partly adsorbed onto clay particles and thereby becomes difficult to capture using conventional means.

Ikeda et al. (Microbes Environ. Vol. 23, No. 2, 159-166, 2008) evaluates the effect of different additives in improving the DNA/RNA extraction yield and quality. It was reported that although skim milk and casein were quite useful in order to improve the yield, residual DNA in skim milk and casein caused contamination problems. Use of RNA was therefore more preferable.

Hoshino and Matsumoto (Soil Biology & Biochemistry 39 (2007) 434-444) suggest using DNA as an adsorption competitor when extracting RNA from clay-rich volcanic ash soil.

US 5,753,466 likewise discloses a method comprising addition of nucleic acids (DNA or RNA) to soil in order to facilitate extraction of microbial DNA.

Barton et al. (J. Microbiol. Meth. 66 (2006) 21-31) discloses the use of the synthetic DNA molecule poly-dIdC as a blocking agent and carrier molecule to increase the yield when extracting DNA from low-biomass carbonate rock. Barton et al. however reported that commercial grade poly-dIdC was contaminated with rRNA sequences, and therefore found it necessary to decontaminate commercial poly-dIdC by means of UV cross-linking in order render it useful as a carrier molecule.

Due to the synthetic nature of the poly-dIdC, however, the use of this constituent (including any decontamination step) as a blocking agent is relatively costly for the purpose of routine DNA/RNA extractions from highly adsorptive samples.

Rockland-Immunochemicals, Inc. provides salmon sperm DNA ready-to-use solution containing single-stranded DNA mecanically shared and heat denaturated to an average size of 100 to 200 base pairs, in a concentraton of 5.0 mg/ml.

Golenberg et al, Nucleic Acids Research, 1996, Vol. 24, No. 24, p. 5026-5033 relates to the effect of highly fragmented DNA on PCR. Fragmentation due to autolysis, bacterial degradation and background spontaneous depurination severely reduces the efficiency of the PCR.

According to Frost and Guggenheim, Oxford University Press, Nucleic Acids Research, 1999, Vol. 27, No. 15, p. e6, depurination of DNA during elution from gels adversely affects its ability to act as a template for PCR.

Dizdaroglu and Simic, Jurnal of Chromatography, 1980, 273-279 discloses isolation of fragments of defined sequence (e.g., 5 bp) from hydrolysates of depurinated herring sperm DNA.

### OBJECT OF THE INVENTION

It is an object of embodiments of the invention to provide a cost-efficient way of improving the yield and quality of DNA/RNA extractions from samples of DNA/RNA adsorbing matrices.

### SUMMARY OF THE INVENTION

It has been found by the present inventors that the DNA/RNA extractions from samples of DNA/RNA adsorbing matrices can be improved in a cost-efficient manner by pre-treating the sample.

So, a first aspect the present invention relates to a method for pre-treating a sample of a DNA/RNA adsorbing matrix suitable for extracting cell-derived (deoxy)ribonucleic acid ("target DNA/RNA") therefrom, cf. claim 1.

A second aspect of the present invention relates to a method for extracting cell-derived (deoxy)ribonucleic acid ("target DNA/RNA") from a DNA/RNA adsorbing matrix, cf. claim 9.

### LEGENDS TO THE FIGURES

Figure 1: Real time PCR curves of samples containing cloned standard *Dehalococcoides* sp. 16S rRNA genes (gray tone) and DNA extracted from the KB-1 culture in presence and absence of sediment and sDNA (Black tone). The quantification is done where the individual curves crosses the line Flouresence=100. The earlier the curve crosses the line the higher concentration of *Dehalococoides* sp.
Figure 2: Sorption isotherms for *E. coli* DNA in 9 different soils/sediments.
Figure 3: Curves showing the efficacy of DNA extraction as measured by qPCR from 4 soil samples of KB-1 and 16 S DNA as a function of the amount of fragmented salmon sperm DNA added in dissolved form to a bead solution in a commercial DNA extraction kit.
Figure 4: Curves showing the efficacy of DNA extraction as measured by qPCR from 4 soil samples of KB-1 and 16 S DNA as a function of the amount of fragmented salmon sperm DNA present in freeze-dried form together with extraction beads in a commercial DNA extraction kit.

### DETAILED DISCLOSURE OF THE INVENTION

### A method for pre-treating a sample

As mentioned above, a first aspect of the present invention relates to a method for pre-treating a sample of a DNA/RNA adsorbing matrix suitable for extracting cell-derived (deoxy)ribonucleic acid ("target DNA/RNA") therefrom, comprising the steps of:
- isolating a sample of a DNA/RNA adsorbing matrix, said sample comprising DNA/RNA-containing cells; and
- bringing the sample into contact with DNA of natural origin in a fragmented form ("fragmented DNA") so as to essentially block said DNA/RNA binding sites of said adsorbing matrix, said fragmented DNA being "unrelated" to the target DNA/RNA of said DNA/RNA-containing cells, wherein
   1) the fragmented DNA provides no response in a polymerase chain reaction (PCR) experiment targeting the 16S rRNA gene with 0.4 µM of each of the primers 341f (SEQ ID NO: 4) and 518r (SEQ ID NO: 5) using 10 ng of the fragmented DNA as a template, where the PCR reaction is run at 95°C for 5 min., followed by 30 cycles of 95°C for 45 sec, 55°C for 30 sec., 72°C for 45 sec., and a final step of 72°C for 7 min, and
   2) the fragmented DNA has a fragment length of up to 300 base pairs (bp) and/ or the fragmented DNA is depurinated.

The overall purpose of the method of the invention is to improve the yield and quality of DNA/RNA extraction from samples of DNA/RNA adsorbing matrices. In many of the most relevant embodiments, the underlying problem resides in the fact that the DNA/RNA adsorbing matrix tends to adsorb a significant quantity of DNA/RNA molecules thereby leaving behind a reduced number of DNA/RNA molecules available for extraction under conventional (mild) conditions. As an example, DNA/RNA extraction from DNA/RNA-containing cells (e.g. bacterial cells) present in soil sample (e.g. low-biomass soil samples, such as clay-rich sub-soil samples) is complicated by the fact that as soon as the DNA/RNA molecules are liberated from the DNA/RNA-containing cells (e.g. by lysis), they have a tendency to become absorbed to mineral particles of the soil sample. The present invention provides a cost-efficient solution to this problem.

In the prior art methods discussed above, nucleic acids have been suggested as a pre-treatment agent, but none of the approaches suggested have utilised fragmented DNA of natural origin as herein described.

It should be understood that present invention may be applicable for pre-treatment of samples of a wide range of DNA/RNA adsorbing matrices.

In the present context, the term "DNA/RNA adsorbing matrix" is intended to mean a material having DNA/RNA adsorbing binding sites, i.e. a matrix which is capable of adsorbing or otherwise binding DNA and/or RNA molecules, e.g. via the phosphor moieties, the sugar moiety and/or the nucleobase moiety of said molecules.

Illustrative examples of such DNA/RNA adsorbing matrices are soil samples, sub-soil samples, sediment samples, seed samples, and concrete samples.

Particularly relevant DNA/RNA adsorbing matrices are soil samples, in particular clay-rich soil samples. Soil is typically described as a mixture of mineral and organic constituents that are in solid, gaseous and aqueous states. Soil particles pack loosely, forming a soil structure with pore spaces or interstices filled with liquid or air.

DNA/RNA extraction from low-biomass samples (e.g. clay-rich sub-soil samples) can in particular benefit from the present invention. Such soil samples are characterised by having a relatively low content of organic matter and therefore leave behind a significant area of the mineral particles available for adsorption of DNA/RNA molecules.

In some embodiments, the organic matter of such "low-biomass" soil samples constitutes less than 5 % by weight, such as less than 1 % by weight, of the sample dried at 60 °C for 24 hours at 50% RH.

In some embodiment, the DNA/RNA extraction is from samples having a fairly low abundance of the relevant DNA/RNA-containing cells. Hence, the present invention is also particularly interesting for samples of the DNA adsorbing matrix which comprises less than 5 x 10⁷, such as less than 1 x 10⁷, DNA-containing cells per g of the sample (the mass of the sample being determined after drying at 60 °C for 24 hours at 50% RH).

In the first step of the method of the invention, a sample of a DNA/RNA adsorbing matrix is isolated.

It is apparent that the sample in question should be of a type which at least potentially comprises DNA/RNA-containing cells of a type which is of interest for the purpose of the DNA/RNA extraction of the target DNA/RNA therefrom.

A sample for the purpose of the pre-treatment step typically comprise between 0.25 and 10 grams dry weight material. After isolation, it might be stored at -20°C or -80°C, and it is typically not treated further prior to the pre-treatment step according to the invention and the subsequent step of extracting the relevant DNA/RNA. In some embodiments of the invention, the fragmented DNA may advantageously be added (see below) to the sample prior to freezing, since DNA/RNA from the DNA/RNA-containing cells might be liberated from the DNA/RNA-containing cells during freezing.

In a second step of the method of the invention, the sample is brought into contact with DNA of natural origin in fragmented form ("fragmented DNA") so as to essentially block said DNA/RNA binding sites of said adsorbing matrix. It is important for the purpose of the subsequent DNA/RNA extraction, that the fragmented DNA is "unrelated" to the target DNA/RNA of the DNA/RNA-containing cells.

The expression "natural origin" implies that the DNA is not of synthetic origin, i.e. that the (native) DNA molecule (before being turned into the fragmented form) is established in a live organism, e.g. a vertebrate, a plant, a mammal, a fungi, a bacterium, a yeast, or the like. Currently preferred examples are vertebrate cells, plant cells, fungi, or yeast, in particular vertebrate cells, such as fish sperm, e.g. salmon sperm or herring sperm, or calf thymus.

Use of DNA of synthetic origin would be detrimental to the concept of the present invention, namely to provide a cost-efficient methodology. As an example, the use of polydIdC has been proposed as a way to overcome the problems of false positives, but the cost of polydIdC is approx. 125 € for 500 µg (2009 prices) and 1-5 mg is needed per gram of DNA/RNA adsorbing material. In contrast hereto, the price of fragmented salmon sperm DNA is approx. 100 € for 50 gram, i.e. 100,000 times less.

The DNA should be present in a fragmented form, hence the term "fragmented DNA". It is essential that the DNA is fragmented because it minimizes the risk of contents of contaminating DNA sequences that will lead to false results (such as false positive results) in down-stream applications. Another advantage is that fragmented DNA provides the opportunity to apply certain silica columns with a cut-of value for DNA/RNA fragments of e.g. >200 bp, thereby rendering it simple to separate the fragmented DNA from the nucleic acids subsequently extracted from the sample.

The DNA is fragmented to such a degree that the fragmented DNA provides no response in a polymerase chain reaction (PCR) experiment targeting the 16S rRNA gene with the primers 341f and 518r described in the Examples section using 10 ng of the fragmented DNA as template. This simple functional test ensures that the DNA is sufficiently fragmented so as to not interfer with subsequent amplification and detection of nucleic acids derived from the matrix.

In one embodiment, the fragmented DNA has a fragment length of up to 300 base pairs (bp), such as a fragment length of up to 250 base pairs (bp), in particular a fragment length of up to 200 base pairs (bp). In other words, it is preferred that the fragmented DNA essentially does not comprise DNA having more than 300 bp.

In certain embodiments of the invention, the fragmented DNA used is further depurinated, *i.e.* the fragmented DNA has been subjected to a treatment where purine bases (adenine and guanine) have been cleaved off from the sugar-phosphate backbone of the DNA molecules. The degree of depurination may vary, but in this particular embodiment it is essential that the fragmented DNA fragments are depurinated to such a degree that they are unable to serve as templates in a PCR.

The step of depurination may be combined with a subsequent heat treatment step and/or a subsequent alkaline treatment step; this results in further fragmentation of the depurinated DNA because the DNA strand is cleaved at the depurinated sites - the main advantage is that the resulting DNA, while still being capable of blocking available sites in e.g. a matrix of clay, will not be visible in an electrophoresis gel.

Various methods are available for the purpose of fragmenting the DNA, e.g. fragmentation by sonication, by exposure to UV light, or by high mechanical shear, but in line with the above also depurination followed by heat or alkaline treatment. It is currently preferred that the DNA is fragmented by sonication, but the various methods for fragmentation may be freely combined with each other. Especially preferred is a combination of any fragmentation method together with depurination followed by heat treatment or alkaline treatment. Especially preferred is defragmentation by means of sonication followed by depurination (cf. below) and heat treatment.

When performing depurination this is preferably performed by acid treatment at a pH below 3.0, but lower values are preferred such as pH values below 2.9, below 2.8, below 2.7, below 2.6, below 2.5, below 2.4, below 2.3, below 2.2, below 2.1, below 2.0, below 1.9, below 1.8, below 1.7, or even below 1.6. pH values at or below 1.5 are especially preferred for the depurination step. The time for the acid treatment will typically be from a few minutes to half an hour.

The heat treatment step following depurination will typically take place at temperatures above 100°C, such as temperatures normally used for autoclaving (*i.e.* temperatures between 110°C and 140°C such as temperatures about 125°C). If alkaline treatment is used after the depurination step, the pH is typically above 9.0, but higher values may be used such as above 9.5, above 9.5, above 9.6, above 9.7, above 9.8, above 9.9 or above 10.0..

In some embodiments, it might be desirable to filter a solution of the fragmented DNA through a size-exclusion column with a cut-off value of approx. 300 bp, such as approx. 250 bp, in particular approx. 200 bp, so as to remove fragmented DNA with a size larger than the desirable length. A silica column may be useful for this purpose. As mentioned above, this step may be unnecessary if the fragmented DNA is depurinated and subjected to heat and/or alkaline treatment.

Moreover, it is important that the fragmented DNA is unrelated to the target DNA/RNA of the DNA/RNA-containing cells. The expression "unrelated" means that the DNA originates from a source that is not related to the target DNA (e.g. using DNA from a eukaryotic source when targeting prokaryotic DNA/RNA and vice versa).

In one currently preferred embodiment, the DNA/RNA-containing cells are bacterial cells, and the fragmented DNA originates from eukaryotic cells, such as vertebrate cells, plant cells, fungi, yeast, in particular vertebrate cells, such as fish sperm, e.g. salmon sperm or herring sperm, or calf thymus.

In another embodiment the DNA/RNA-containing cells originates from eukaryotic cell material such as vertebrate cells, plant cells, fungi, or yeast, and the fragmented DNA originates from prokaryotic cells, such as Escherichia coli.

Depending on the ability of the material to bind (deoxy)ribonucleic acids, the fragmented DNA is typically applied in an amount of 0.5-50 mg per g of the sample, e.g. in an amount of 1-20 mg per g of the sample.

The fragmented DNA is typically dissolved in DNAse/RNase free water before use. In other interesting embodiments, it is dissolved directly in a liquid lysis or extraction buffer or a bead solution from a conventional kit, e.g. a commercial kit (see also further below). As an alternative, the DNA may be present in the kit in dried form (e.g. freeze-dried) together with the dry beads.

In most practical embodiments, the fragmented DNA is dissolved in either water or an extraction buffer/bead solution is added to the sample (or, in the case the fragmented DNA is present with the beads, the extraction buffer is added to the bead/fragmented DNA mixture), and the mixture is vortexed to ensure contact between sorption sites of the DNA/RNA absorbing matrix and the fragmented DNA. The sample should be incubated at room temperature for a couple of minutes, or more. The sample is then typically ready for subsequent DNA/RNA extraction (see below).

### A method of extracting DNA/RNA

As mentioned above, a second aspect of the present invention relates to a method of extracting cell-derived (deoxy)ribonucleic acid ("target DNA/RNA") from a DNA/RNA adsorbing matrix, said method comprising the steps of:
- pre-treating a sample of a DNA/RNA adsorbing matrix in accordance with the above-mentioned pre-treatment method, wherein said sample comprises DNA/RNA-containing cells;
- liberating target DNA/RNA from said DNA/RNA-containing cells and extracting said target DNA/RNA from said sample.

In the method of extracting DNA/RNA, the first step is as described further above.

With respect to the second step, conventional methods for liberating target DNA/RNA from the DNA/RNA-containing cells (e.g. by lysis, freeze-drying, etc.) and for extracting the target DNA/RNA from the sample are typically used.

Typically DNA/RNA is extracted from biological cells by using a cell lysing buffer in combination with mechanical disruption and/or sonication. Sometimes when complex biological samples are extracted, proteinase K treatment is used to break down connective tissue.

In some currently interesting embodiments, the method of extraction includes the following steps.
- isolating a sample of a DNA/RNA adsorbing matrix, said sample comprising DNA/RNA-containing cells;
- providing the fragmented DNA (in particular sonicated DNA) in a solution (e.g. in water or a suitable buffer), preferably in a concentration of 1-30 mg/mL.
- bringing the sample into contact with DNA of natural origin in a fragmented form ("fragmented DNA") so as to essentially block said DNA/RNA binding sites of said adsorbing matrix, said fragmented DNA being "unrelated" to the target DNA/RNA of said DNA/RNA-containing cells. Depending on the ability of the material to bind (deoxy)ribonucleic acids, the fragmented DNA is typically added to the sample to a final concentration of 1-20 mg/g of the sample.
- In order to ensure a certain distribution of the sDNA into the material the final volume of liquid is typically a low as possible, e.g. typically approx. 2 mL per g of the sample.
- The sample is shaken vigorously (e.g. vortexed).
- The sample is freeze-dried in order to remove the liquid phase.
- (Deoxy)ribonucleic acids (DNA or RNA) are extracted from the sample using conventional methods.

Alternative embodiments of DNAR/RNA extraction, which are exemplified in Example 4, entail use of the fragmented DNA simultaneously with the step of liberating and extracting DNA/RNA, meaning that pretreating the matrix and liberation and extraction of DNA/RNA is performed in one single step. One way of achieving this is accomplished in embodiments, where the fragmented DNA used for pre-treating is part of an extraction/lysis buffer used for liberating/extracting the target DNA/RNA. As shown in Example 4(A), this mode of the invention provides for satisfactory results in terms of yield of extracted nucleic acids.

An alternative way of accomplishing this one-step pre-treatment and liberation/extraction is by having the fragmented DNA present in a reaction vessel where liberation and extraction of DNA/RNA is performed by addition of an extraction/lysis buffer and the DNA/RNA adsorbing matrix. Advantageously, the fragmented DNA is present in the vessel in dry from, such as in freeze-dried form. This particular embodiment is especially useful when liberation and extraction entails physical disruption of the DNA/RNA-containing cells. Many commercial kits can thus be modified so as to utilise such reaction vessels at least comprising the DNA/RNA adsorbing matrix, a lysis/extraction buffer, and fragmented DNA, and then subjecting the reaction vessel to sonication, exposure to UV light, or by high mechanical shear. The vessel may further comprise beads which assist in the physical disruption of the cells, as is the case for many commercial kits for DNA extraction.

### Kits

The present disclosure also enables the provision kits for the extraction of DNA or RNA, in particular in accordance with the method described above.

Hence, one enabled kit is for extracting cell-derived (deoxy)ribonucleic acids from a sample of a DNA/RNA adsorbing matrix comprising DNA/RNA-containing cells, said kit comprising a closed container (e.g. a flask with a stopper or screw-cap, a vial, or the like) having included therein a solution of DNA of natural origin in a fragmented form ("fragmented DNA") in a concentration of 1-30 mg/mL, said fragmented DNA having a fragment length of up to 300 base pairs (bp), such as a fragment length of up to 250 base pairs (bp), in particular a fragment length of up to 200 base pairs (bp).

The kit is intended for pre-treatment of samples intended for DNA/RNA extraction and can therefore in some interesting embodiments be construed as an add-on component for existing commercial DNA/RNA extraction kits.

In one interesting embodiment of such a kit the container having included therein the solution of DNA is a lysis buffer, i.e. the solution useful for blocking the DNA/RNA adsorbing binding sites of the adsorbing matrix is at the same time useful for lysing the DNA/RNA-containing cells.

The kit may include further reagents and components, The a lysis buffer, an extraction buffer, a combined lysis/extraction buffer. The fragmented DNA can be dissolved in a combined lysis/extraction buffer. The kit may further comprise a reagent for precipitation of proteins, salts, etc. for the preparation of the target DNA/RNA extract for collection via a silica column.

The kit may further comprise a filter (e.g. a silica column) having a cut-off value in the range of 200-500 bp for DNA fragments. In this way, residuals of the fragmented DNA will be washed through the filter, while the target DNA/RNA will be captured in the filter for further clean-up and subsequent elution.

An alternative kit for extracting cell-derived (deoxy)ribonucleic acids from a sample of a DNA/RNA adsorbing matrix comprising DNA/RNA-containing cells, comprises a closed container having included therein a dried DNA of natural origin in a fragmented form ("fragmented DNA"), said fragmented DNA having a fragment length of up to 300 base pairs (bp). The dried DNA is typically freeze-dried.

As explained above, such a kit is typically one wherein the dried DNA is present in a reaction vessel, optionally in admixture with beads. The kit may further comprise a lysis buffer. and/or which further comprises a silica column having a cut-off value in the range of 200-500 bp.

As mentioned above, the invention-enabled kit can be included in commercial kits intended for the extraction of DNA and/or RNA from adsorbing materials. Among the kits currently available on the market are kits intended for analysis of soils, e.g.: FastSoil DNA (MP), FastSoil RNA (MP), RNA PowerSoil (MoBio), UltraClean Soil (MoBio), PowerSoil (MoBio), PowerMax (MoBio), UltraClean Mega Soil (MoBio) and SoilMaster (Epicentere). These kits are marketed to microbial ecologist, forensic laboratories and ancient DNA laboratories.

The invention-enabled kit can further be used in a variety of different kits intended for extraction of (deoxy)ribonucleic acid from other matrices than soil, this includes but is not limited to RNaEasy (Qiagen), WaterMaster (Epicentre), UltraClean Water kit, PowerPlant (MoBio); and FastDNA spin kit for plants and animal tissues (MP).

In all the embodiments of the kit described above, it is particularly preferred that the fragmented DNA is depurinated, cf. the detailed discussion of this feature above.

### EXAMPLES

### Materials and Methods

### Polymerase chain reaction (PCR) experiment targeting the 16S rRNA gene with the primers 341f and 518r

A polymerase chain reaction (PCR) experiment targeting the 16S rRNA gene with the primers 341f and 518r was conducted as described by Muyzer et al. (Muyzer, G., Dewaal, E. C., Uitterlinden, A. G. (1993). Profiling of complex microbial populations by denaturing gradient gel-electrophoresis analysis of polymerase chain reaction-amplified genes coding for 16S ribosomal-RNA. Appl. Environ. Microbiol. 59:695-700) using 10 ng of a sample of fragmented DNA as the template.

### Example 1 - Experiment with extraction of the dehalogenating bacterial culture KB-1

### Materials:

1. Ultraclean MilliQ water.
2. Sonicated deoxyribonucleic acids (sDNA) from Fluka analytical #31149.
3. Clay rich groundwater sediment taken from 8 m below surface from Vasbyvej, Denmark.
4. KB-1 culture containing *Dehalococcoides sp.* from Sirem, Canada.
5. UltraClean soil DNA extraction kit from MoBio, Ca, USA # 12800-50.
6. Real time PCR ingredients for quantifying *Dehalococcoides* sp.
   - DyNAmo - SybrGreen qPCR kit from FinnZymes, Finland #F-400.
   - Dehalococcoides sp. specific primers. forward: 5'-GGGAGTATCGACCCTCTC-3' (SEQ ID NO: 1) and reverse: 5'-GGATTAGCTCCAGTTCACACT-3' (SEQ ID NO: 2) obtained from MWG, Germany.
   - Bovine serum albumine (BSA), New England Biolabs, USA.
   - PCR grade water.

### Methods:

1. sDNA was dissolved in ultra clean MilliQ water to a final concentration of 10 mg/mL.
2. Samples were prepared in micro centrifuge tubes as follows: 200 mg sediment, 200 µl KB-1 culture, sDNA and ultra clean MilliQ water to a final volume of 1 mL and concentrations of sDNA of 10, 100, 1000, and 5000 µg/g of sediment. The individual samples in this experiment were set up in triplicate.
3. Samples were vortexed.
4. Samples were frozen at -80 °C.
5. Samples were freeze dried for 13 hours.
6. DNA was extracted from the samples using the UltraClean soil DNA extraction kit from MoBio following the manufacturer's instructions.
7. The number of *Dehalococcoides* sp. Specific 16S rRNA genes were quantified using real time PCR:
   - Mastermix for 20 µL reactions: 10 µL DyNAmo polymerase mix, 0.4 µM of each primer, 1 µg/µL BSA, 1 µL DNA extract, and water was added to a final volume of 20 µL.
   - Reaction conditions: Real-time PCR was performed on an Icycler real time PCR machine from BioRad, CA, USA. An initial 15 min. step at 95°C was performed for enzyme activation, followed be 50 cycles of 95°C for 45 sec., 55°C for 30 sec., 72°C for 1 min., and 82°C for 30 sec. Fluorescence was measured after each cycle at 82°C. The PCR was ended by a 72°C step for 7 min. After the PCR a melting curve analysis was performed as a 0.5°C increase in temperature over an interval from 55°C to 98°C and fluorescence was measured after each increase.

   Standard concentrations of *Dehalococcoides* sp. 16S rRNA genes was prepared with plasmid DNA with the full length *Dehalococcoides* sp. 16S rRNA gene cloned into it. Calculations of the exact copy numbers was done as described in Park and Crowley (2005) (Park, J. W., Crowley, D. E. 2005. Normalization of soil DNA extraction for accurate quantification real-time PCR and of target genes by DGGE. Biotechniques, 38:579-586). The concentrations 10³, 10⁴, 10⁵, and 10⁶ genes µl⁻¹ was used in real time PCR as described above in order to construct a standard curve.

### Results:

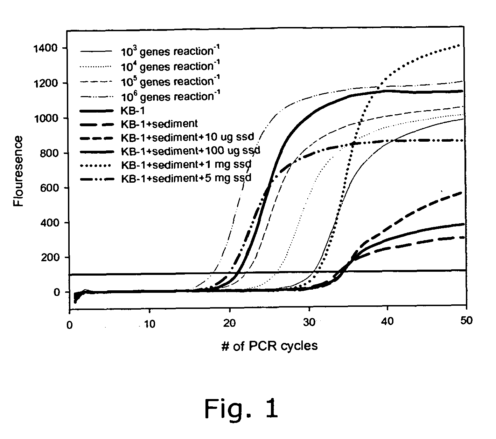

From the results given in Fig. 1 it is very clear that the addition of fragmented salmon sperm DNA has a great impact on the target DNA extraction efficiency from the samples containing sediment. The yield of *Dehalococcoides* sp. 16S rRNA genes was a 1000-fold lower for the samples containing KB-1 culture and 200 mg of sediment compared to the samples containing only KB-1 culture. Addition of low amounts of fragmented salmon sperm DNA had no effect and only when adding as much as 1 mg/g sediment we observed a slight effect. With addition of 5 mg/g sediment we were actually able to retrieve more *Dehalococcoides* sp. 16S rRNA genes than from the KB-1 culture alone. This was probably due to the fact that the KB-1 culture comes with a little bit of inorganic material which also has the potential to adsorb DNA. Another explanation is that the sediment used in this experiment was taken from a contaminated site and therefore it could potentially contain a small number of *Dehalococcoides* sp., which was then co-extracted. The conclusion from this experiment is that it is beyond doubt that fragmented salmon sperm DNA has a great impact on the extraction efficiency of DNA from highly clayey sediment.

### Example 2 - Verification of the fragmented DNA being free of contaminating DNA sequences

### Rationale:

This experiment was done in order to verify whether the sDNA contained any prokaryotic contaminating DNA sequences. We were using a PCR approach targeting a very small and universal fragment of the 16S rRNA gene. Since this approach is targeting such a short fragment (∼180 bp) and since this the regions where the primers binds are thought to be universal for all prokaryotes it is ideal for this test.

### Materials:

1. Ultraclean MilliQ water.
2. Sonicated deoxyribonucleic acids (sDNA) from Fluka analytical #31149.
3. illustra™ puReTaq Ready-to-go PCR beads (GE Healthcare, Buckinghamshire, UK).
4. PCR primers forward:reverse: obtained from MWG, Germany.
5. PCR grade water.
6. 1.5% (w/v) agarose gel (MoBio, CA, USA) prepared in 1xTAE buffer.
7. Regular equipment for running and visualizing agarose gels.

### Methods:

1. sDNA was dissolved in Ultraclean MilliQ water to a final concentration of 10 µg/mL.
2. PCR was setup in 25 µL reactions with primers in concentrations of 0.4 µM and with addition of 10 ng of sDNA as template in one reaction and 10 ng of plasmid DNA with a *Dehalococcoides* sp. 16S rRNA gene insert in a second reaction as a positive control and at last a reaction containing no DNA as template intended for a negative control.
3. The PCR was done on an Icycler using the following conditions: 95°C for 5 min., 30 cycles of 95°C for 45 sec, 55°C for 30 sec., 72°C for 45 sec., and a final step of 72°C for 7 min.
4. 5 µL of each of the PCR products were loaded on an agarose gel and run along with an 100 bp ladder and 2 ng of sDNA, all in individual lanes. The Agarose gel was run for 45 min at 120 V. The gel was stained with EtBr and visualized in a GelDoc apparatus.

### Example 3 - Sorption of DNA to different soil types

### Materials:

1. ³H-labelled DNA extracted from *E.coli.* This was produced by growing *E.coli* in presence of ³H-labelled Thymidine (Sigma-Aldrich, USA) in LB-media. DNA was then extracted with Ultraclean Microbial DNA extraction kit (MoBio, CA, USA)
2. 0.1 M Tris-buffer.
3. Nine different Gamma radio sterilized soils
   - Vasbyvej clayey sampled 8 m below surface, Hedehusene, Denmark.
   - Vasbyvej sandy sampled 6 m below surface, Hedehusene, Denmark.
   - Sj.O A, Sjællands Odde, topsoil, Denmark.
   - Sj.O B, Sjællands Odde, sub soil sampled 50 cm below surface, Denmark.
   - Hojbakke gård A, Topsoil, Høje Tåstrup, Denmark.
   - Acidic soil, Rice pad sediment, Vietnam.
   - Jyndevad A, Topsoil, Jyndevad, Denmark.
   - Jyndevad B, Sub soil sampled 50 cm below surface, Jyndevad, Denmark.
   - Alluvial soil, Rice pad sediment, Vietnam.
4. Standard equipment for quantifying scintillation vials

### Methods:

1. Standard sorption experiments were setup on all of the 9 soils.
   - 0.2 g of soil/sediment was placed in glass tubes along with 3 mL of 0.1 M tris buffer.
   - In order to study the sorption capacity of each of the 9 soils, four different concentrations (1, 10, 100, and 1000 µg/g soil) of *E. coli* ³H-labelled DNA was added enabling us to study sorption isotherms.
   - In order to study the effect of sDNA on the sorption we added 100, 1000, and 5000 µg of sDNA g⁻¹ of soil to individual sets of glasses, enabling us to study sorption isotherms for each of the sDNA concentration in 5 of the 9 different soils. These 5 five soils, were the soils in which we had observed a very high sorption potential.
2. The glass tubes were rotated end over end for 24 hours.
3. The tubes were centrifuged at 2500xg for 10 min and 1 mL supernatant was transferred to a scintillation vial containing 10 mL of scintillation fluid.
4. Scintillation vials were quantified for 10 min. in a scintillation counter.
5. Sorption data was fitted to Langmuir kinetics where possible.

### Results:

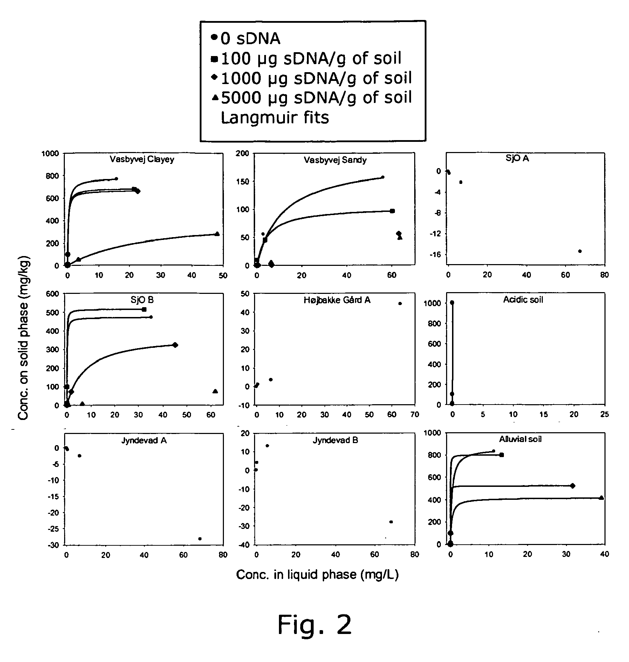

It is clear from the results shown in Figure 2, that sorption of DNA to soil or sediment is very different based on the origin of the soil/sediment. In 5 of 9 soils we observed a very strong sorption of DNA, while in the remaining 4 soils we observed no sorption at all. A general trend was that the strong sorption was observed in soils with very low amounts of organic matter, either sampled from deep sub soil layers or from other locations poor in organic matter.

### Example 4 - Alternative methods for blocking binding sites in soil with sDNA

### (A): Use of dissolved, fragmented DNA in the DNA extraction

4 soil specimens were used:
"Fyn Sediment", from the Danish island Fuen
"Sj. Odde A horizon", from a location in the north west of Danish Island Zealand
"Sj. Odde B horizon", from a location in the north west of Danish Island Zealand
"Hellerup beach", from a beach just north of Copenhagen.

24 x 200 mg of each soil sample was transferred to individual clean bead tubes, providing a total of 92 samples.

In 12 samples from each soil specimen 200 µl KB-1 solution was added (conc. 4 x 10⁸ cells ml⁻¹ of which approx. 10% is *Dehalococcoides* sp.). In order to have equally amounts of liquid in the samples, 200 µl H₂O was added to the remaining 12 samples from each soil specimen.

Fragmented salmon sperm (termed "G1" in the following) was dissolved in a bead solution to a concentration of 10 mg/ml. The pH in the bead solution was measured before G1 was added after which G1 containing solution was adjusted according to the original bead solution pH. Then the solution was sterile filtrated on a 0.22 µm filter.

From this solution, 4 concentrations were prepared: 10 mg/ml, 2 mg/ml, 0.2 mg/ml and 0 mg/ml.

DNA extraction from cells in 3 soil samples with added KB-1 and 3 without added KB-1 from each sample site was performed using each of the G1 Bead solutions, *i.e.* 3 + 3 samples were DNA extracted with 10, 2, 0.2 and 0 mg/ml G1 Bead solution resulting in a total of 4 × 24 DNA extraction experiments.

Extractions were otherwise performed using the Ultraclean Fecal DNA isolation kit (MoBio) in accordance with the manufacturer's instructions, except that 45 sec of bead-beating in an FastPrep machine replaced 10 min in an vortex adapter.

Quantification of extraction yield was done by real time qPCR with Maxima™ SYBR Green qPCR Master Mix (2X) (Fermentas) as polymerase.

The primes used in the qPCR were:
**For KB1:**
   Forward primer (774): 5'- GGG AGT ATC GAC CCT CTC -3' (SEQ ID NO: 1)
   Reverse primer (1212): 5'- GGA TTA GCT CCA GTT CAC -3' (SEQ ID NO: 3)
**For 16 S:**
   Forward primer (341f): 5'- CCT ACG GGA GGC AGC -3' (SEQ ID NO: 4)
   Reverse primer (518r): 5'- ATT ACC GCG GCT GCT -3' (SEQ ID NO: 5)

### (B): Use of freeze-dried, fragmented DNA in the DNA extraction

The same 4 soil specimens as in (A) where used.

G1 was dissolved in milliq water and the pH adjusted to approx. 7, and then the solution was sterile filtrated with Ultraclean Water DNA, Water filters from Mo-Bio.

As in (A), 4 concentrations were prepared: 0 mg/ml, 0.2 mg/ml, 2 mg/ml and 10 mg/ml.

500 µl was added from these concentrations to bead tubes and subsequently freeze-dried; this resulted in 24 bead tubes with 5 mg of freeze-dried G1, 24 bead tubes with 1 mg of freeze-dried G1, 24 bead tubes with 0.1 mg of freeze-dried G1, and 24 tubes with 0 mg G1.

24 samples of 200 mg of each soil specimen were transferred to the bead tubes with freeze-dried G1, resulting in 96 samples in total.

In 12 samples from each soil specimen, 200 pi of KB-11 solution (conc. 4x10⁸ cells ml⁻¹, *i.e.* approx. 10% Dehalococcoides) was added. In the last 12 samples from each soil specimen, 200 µl H₂O was added in order to have equal amounts of liquid in all samples.

DNA extraction from cells in 3 soil samples with added KB-1 and 3 without added KB-1 from each sample site was performed with each of the G1 Bead solutions, *i.e.* 3 + 3 samples were DNA extracted with 10, 2, 0.2 and 0 mg/ml G1 Bead solution resulting in a total of 4 x 24 DNA extraction experiments.

Extractions were otherwise performed using the Ultraclean Fecal DNA isolation kit (MoBio) in accordance with the manufacturer's instructions, except that 45 sec of bead-beating in an FastPrep machine replaced of 10 min in a vortex adapter.

The quantification of the DNA was performed by real time qPCR with Maxima™ SYBR Green qPCR Master Mix (2X) (Fermentas) as polymerase.

The primes used for KB-1 and for 16 S were the same as in (A)

Chemical and physical Characterization of the soil specimens used in (A) and (B):

| | pH | Ca | Mg | Na | K | P | N | C | CaCO₃ | Clay | Silt | Sand |
|---|---|---|---|---|---|---|---|---|---|---|---|---|
| | | cmol(+)/kg | | | | mg PO₄-P/kg | % | | | | | |
| Sj. Odde A | 7.2 | 9.19 | 0.59 | 0.1 | 0.39 | 44.3 | 0.14 | 1.25 | 0.4 | 19 | 18 | 63 |
| Sj. Odde B | 7.6 | 16.55 | 1.76 | 0.21 | 0.31 | 0.5 | 0.05 | 0.3 | 0.3 | 31 | 26 | 43 |

| | pH H₂O | pH CaCl₂ | Carbon | Coarse sand | Clay | Silt | Fine Sand | CEC | Ca | Mg | K | Na | BS |
|---|---|---|---|---|---|---|---|---|---|---|---|---|---|
| | | | % | % | | | | cmol(+)/kg | | | | | % |
| Fyn Sediment | 8.55 | 7.925 | ∼ 0 | 7.4 | 22.8 | 20.8 | 48.9 | 4.0886 | 22.866 | 1.396 | 0.244 | 0.089 | 100% |

There is no available characterization for the Hellerup Beach sample, which is composed of sandy soil from a beach north of Copenhagen.

### Results

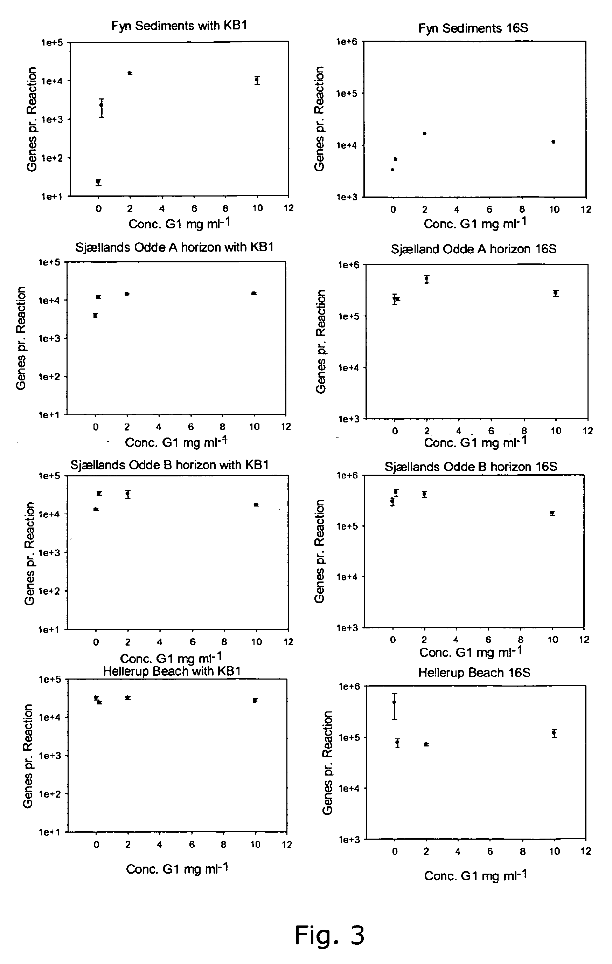

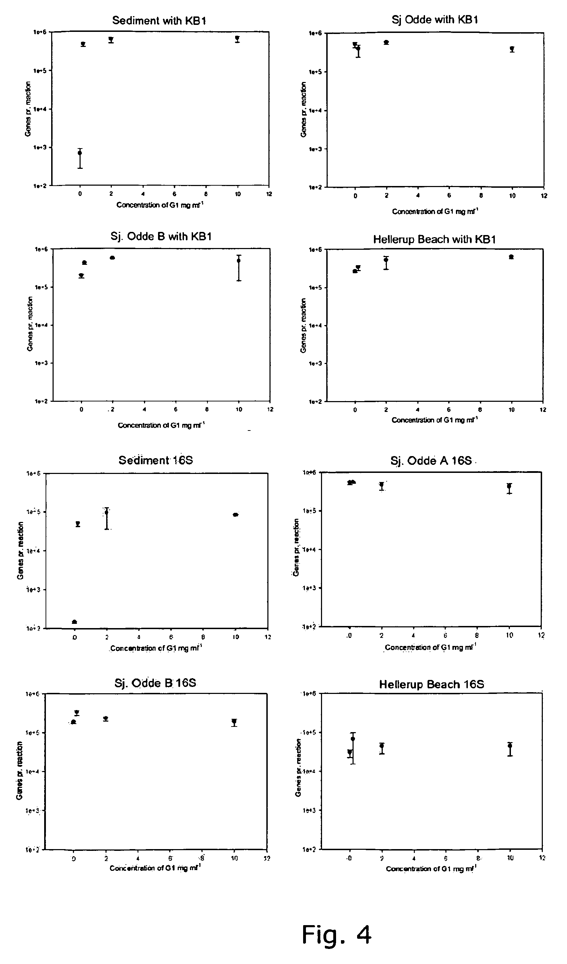

As appear from the data set forth in Figs. 3 and 4, the addition of G3 in increasing amounts improves the yield of amplified KB-1 and 16s DNA in the soil samples from Fuen and Sj. Odde. In analogy with the findings of Example 1 the most marked increase is in the specimens from Fuen, where the soil is relatively rich in clay. For the soil specimen taken from Hellerup Strand, the addition of G3 on the other hand does not have any marked effects, which is in good agreement with the fact that this specimen is from a sandy beach.

These experiments thus verify that treatment with fragmented DNA can be performed by adding the fragmented DNA directly to a bead solution from a conventional DNA extraction kit, either in solution or by having the fragmented DNA present in freeze-dried from in the beads.

### Example 5 - further neutralization of fragmented DNA

In this example it is demonstrated that the nucleic acids in solution may be modified ("neutralized") so that they cannot be amplified in PCR, visualized in a gel or used as target in other downstream molecular assays but nevertheless still act as DNA in terms of priming binding sites on sorbtive matrices like clay soil

### Theory

Depurination of DNA by decreasing the pH in the DNA containing solution to ∼1 for 15 min causes the purines adenine and guanine to be removed from the deoxyribose sugar by hydrolysis of the beta-N-glycosidic link between them and they are thereby replaced by an hydroxyl (-OH) group. If the resulting depurinated DNA is subsequently denatured either by alkaline treatment or by heat, the DNA molecule will be cleaved at the apurinic sites.

### Experiment 1

### Method

Sufficient HCl was added to a sterile filtered 10 mg/ml solution of fragmented DNA (prepared as described above) to decrease pH in the solution to 0.7, 1.5, 3, and 5, respective. The pH was measured with a pH meter. The high concentration of DNA will precipitate at these pH values. After 15 min of stirring, the solutions were carefully heated to 80°C, which causes the DNA to denature and become dissolved again. The pH was hereafter carefully raised to pH~7, and the DNA solutions were autoclaved (15 min at 125°C).

### Results

Standard agarose gel electrophoresis demonstrated that the characteristics of the nucleotides changed as a result of acid treatment at pH 0.7 and 1.5. At pH 3 and 5 we did not observe any changes. The pattern of the acid treated nucleotides was a long smear over the entire gel, compared to the untreated nucleotides that revealed a tight band around 50-150 bp fragment length. This pattern change occurs because adenine and guanine bases have been removed from the nucleotide and thereby changes its electrophoretic properties.

After autoclaving, however, the nucleotides could no longer be visualized on the agarose gel. This was caused by the cleavage of the single stranded nucleotides at the apurinic sites.

### Experiment 2

### Method

Genomic DNA from a bacterial pure culture containing the functional gene *tfdA* was mixed into 10 mg/ml fragmented salmon sperm DNA solution. The DNA solution was hereafter acid treated at pH 1 as described above and afterwards it was autoclaved. 500 µl acid treated and autoclaved DNA solution, was added to MoBio bead tubes together with 200 mg of chemical pure sea sand (baked over night at 300°C to remove DNA residues). Also, tubes were prepared with 500 µl DNA solution that had not been acid treated and autoclaved. The tubes were freeze dried and DNA was extracted from the sea sand using the Ultra Clean Soil DNA kit from Mobio. In order to investigate the effect of acid treatment and autoclaving in terms of the DNA being able to amplify afterwards, the DNA extracts were used as template for real-time PCR with the *tfdA* gene as the target.

### Results

The real-time PCR result for the sample with DNA that had not been acid treated and autoclaved was high with a ct value of ∼18 corresponding to an initial gene content in the extract of ∼4×10⁷ µl⁻¹. In the samples with DNA that had been acid treated and autoclaved there was no amplification meaning that the DNA had been modified so that it could not serve as a template in PCR.

### Experiment 3

This experiment was performed in order to investigate whether the acid treated and autoclaved fragmented salmon sperm DNA still had the ability to block binding sites in clay material and thereby enhance DNA extraction efficiency.

### Method

500 µl 10 mg/ml acid treated and autoclaved fragmented salmon sperm DNA was added to 200 mg sediment samples along with 200 pi *Dehalococcoides* sp. containing culture. For comparison a set of samples were prepared with water instead of fragmented salmon sperm DNA. Seven different soils were included in this experiment. The samples were freeze dried and DNA was extracted using the UltraClean Soil DNA kit from MoBio. The extracts were used for template in real time PCR targeting a *Dehalococcoides* sp. specific region in the 16S. As a control a sample with 200 mg of chemical pure sea sand (baked over night at 300°C to remove DNA residues) and 200 µl KB-1 culture was also prepared.

### Result

The depurinated and autoclaved DNA blocked the binding sites with the same efficiency as the original DNA. The experiment was carried out with 7 selected soils and in five out of the seven soils the addition of acid treated and autoclaved salmon sperm DNA had a great effect and increased the extraction yield ×10-×1000 compared to the sediment samples without addition of salmon sperm DNA. In the last two soil samples there was no difference between the sample with and without salmon sperm DNA addition, but also there was no difference between the sediment samples and the control sample with chemical pure sea sand.

## Claims

1. A method for pre-treating a sample of a DNA/RNA adsorbing matrix suitable for extracting cell-derived (deoxy)ribonucleic acid ("target DNA/RNA") therefrom, comprising the steps of:
a. isolating a sample of a DNA/RNA adsorbing matrix, said sample comprising DNA/RNA-containing cells; and
b. bringing the sample into contact with DNA of natural origin in a fragmented form ("fragmented DNA") so as to essentially block said DNA/RNA adsorbing binding sites of said adsorbing matrix, said fragmented DNA being "unrelated" to the target DNA/RNA of said DNA/RNA-containing cells,
wherein
1) the fragmented DNA provides no response in a polymerase chain reaction (PCR) experiment targeting the 16S rRNA gene with 0.4 µM of each of the primers 341f according to SEQ ID NO: 4 and 518r according to SEQ ID: NO: 5 using 10 ng of the fragmented DNA as a template, where the PCR reaction is run at 95°C for 5 min., followed by 30 cycles of 95°C for 45 sec, 55°C for 30 sec., 72°C for 45 sec., and a final step of 72°C for 7 min, and
2) the fragmented DNA has a fragment length of up to 300 base pairs (bp) and/or the fragmented DNA is depurinated.

2. The method according to any one of the preceding claims, wherein the fragmented DNA is depurinated.

3. The method according to claim 2, wherein depurination is accomplished by acid treatment at a pH below 2.0.

4. The method according to any one of the preceding claims, wherein the DNA of natural origin is fragmented by sonication and/or by exposure to UV light and/or or by high mechanical shear and/or by depurination combined with heat treatment or alkaline treatment.

5. The method according to claim 3, wherein the heat treatment has been performed at a temperature above 100°C and/or wherein the alkaline treatment has been performed at a pH above 9.

6. The method according to any one of the preceding claims, wherein the DNA/RNA-containing cells are bacterial cells, and the fragmented DNA originates from eukaryotic cells, such as vertebrate cells.

7. The method according to any one of the preceding claims, wherein the fragmented DNA is typically applied in an amount of 0.5-50 mg per g of the sample.

8. The method according to any one of the preceding claims, wherein the sample of a DNA/RNA adsorbing matrix is a soil sample.

9. A method of extracting cell-derived (deoxy)ribonucleic acid ("target DNA/RNA") from a DNA/RNA adsorbing matrix, said method comprising the steps of:
a. pre-treating a sample of a DNA/RNA adsorbing matrix in accordance with the method of any one of claims 1-8, wherein said sample comprises DNA/RNA-containing cells;
b. liberating target DNA/RNA from said DNA/RNA-containing cells and extracting said target DNA/RNA from said sample,
wherein steps a and b are optionally performed in one step.

10. The method according to claim 9, wherein the fragmented DNA used for pre-treating is part of an extraction/lysis buffer used for liberating/extracting the target DNA/RNA, or wherein the fragmented DNA is present in a reaction vessel where liberation and extraction of DNA/RNA is performed by addition of an extraction/lysis buffer and the DNA/RNA adsorbing matrix, wherein the fragmented DNA may be present in the vessel in dry form, such as in freeze-dried form.

11. The method according to claim 9 or 10, wherein liberation and extraction entails physical disruption of the DNA/RNA-containing cells, wherein said physical disruption may entail subjecting a reaction vessel at least comprising the DNA/RNA adsorbing matrix, a lysis/extraction buffer, and fragmented DNA to any one of sonication, exposure to UV light, or high mechanical shear.

12. The method according to claim 11, where the vessel further comprises beads which assist in the physical disruption of the cells.

## Patentansprüche

1. Verfahren zum Vorbehandeln einer Probe einer DNA/RNA-adsorbierenden Matrix, die zum Extrahieren von aus einer Zelle stammender (Desoxy-)Ribonukleinsäure ("Ziel-DNA/RNA") daraus geeignet ist, welches die folgenden Schritte umfasst:
a. Isolieren einer Probe einer DNA/RNA-adsorbierenden Matrix, wobei die Probe DNA/RNAenthaltende Zellen umfasst; und
b. In-Kontakt-bringen der Probe mit DNA natürlicher Herkunft in einer fragmentierten Form ("fragmentierte DNA"), um damit DNA/RNA-adsorbierende Bindungsstellen der adsorbierenden Matrix im Wesentlichen zu blockieren, wobei fragmentierte DNA nicht mit der Ziel-DNA/RNA der DNA/RNA-enthaltenden Zellen verwandt ist,
wobei
1) die fragmentierte DNA keine Reaktion in einem Polymerasekettenreaktionsversuch (PCR-Versuch) liefert, die auf das 16S-rRNA-Gen abzielt, mit 0,4 µM jedes der Primer 341f nach SEQ ID NR. 4 und 518r nach SEQ ID NR. 5 unter Verwendung von 10 ng der fragmentierten DNA als Matrize, wobei die PCR-Reaktion für 5 Min. bei 95 °C durchgeführt wird, gefolgt von 30 Zyklen von jeweils 45 s bei 95 °C, 30 s bei 55 °C, 45 s bei 72 °C, und einem abschließenden Schritt von 7 Min. bei 72 °C, und
2) die fragmentierte DNA eine Fragmentlänge von bis zu 300 Basenpaaren (bp) hat und/oder die fragmentierte DNA depuriniert ist.

2. Verfahren nach einem der vorhergehenden Ansprüche, wobei die fragmentierte DNA depuriniert ist.

3. Verfahren nach Anspruch 2, wobei die Depurinierung durch Säurebehandlung bei einem pH-Wert unter 2,0 erreicht wird.

4. Verfahren nach einem der vorhergehenden Ansprüche, wobei die DNA natürlicher Herkunft durch Beschallung und/oder durch Exposition gegenüber UV-Licht und/oder durch starke mechanische Scherung und/oder durch Depurinierung in Kombination mit Wärmebehandlung oder alkalischer Behandlung fragmentiert ist.

5. Verfahren nach Anspruch 3, wobei die Wärmebehandlung bei einer Temperatur über 100 °C durchgeführt worden ist und/oder die alkalische Behandlung bei einem pH-Wert über 9 durchgeführt worden ist.

6. Verfahren nach einem der vorhergehenden Ansprüche, wobei die DNA/RNA-enthaltenden Zellen Bakterienzellen sind und die fragmentierte DNA aus eukaryontischen Zellen, wie zum Beispiel aus Wirbeltierzellen, stammt.

7. Verfahren nach einem der vorhergehenden Ansprüche, wobei die fragmentierte DNA typischerweise in einer Menge von 0,5-50 mg pro g Probe aufgetragen wird.

8. Verfahren nach einem der vorhergehenden Ansprüche, wobei die Probe einer DNA/RNA-adsorbierenden Matrix eine Bodenprobe ist.

9. Verfahren des Extrahierens von aus einer Zelle stammender (Desoxy-)Ribonukleinsäure ("Ziel-DNA/RNA") aus einer DNA/RNA-adsorbierenden Matrix, wobei das Verfahren die folgenden Schritte umfasst:
a. Vorbehandeln einer Probe einer DNA/RNA-adsorbierenden Matrix nach dem Verfahren nach einem der Ansprüche 1-8, wobei die Probe DNA/RNAenthaltende Zellen umfasst;
b. Befreien von Ziel-DNA/RNA aus den DNA/RNAenthaltenden Zellen und Extrahieren der Ziel-DNA/RNA aus der Probe,
wobei Schritt a und b wahlweise in einem Schritt durchgeführt werden.

10. Verfahren nach Anspruch 9, wobei die zum Vorbehandeln verwendete fragmentierte DNA Teil eines Extraktions-/Lysepuffers ist, der zum Befreien/Extrahieren der Ziel-DNA/RNA verwendet wird, oder wobei die fragmentierte DNA sich in einem Reaktionsgefäß befindet, wo Befreiung und Extraktion von DNA/RNA durch Zugabe eines Extraktions-/Lysepuffers und der DNA/RNA-adsorbierenden Matrix durchgeführt werden, wobei die fragmentierte DNA in dem Gefäß in trockener Form vorhanden sein kann, beispielsweise in gefriergetrockneter Form.

11. Verfahren nach Anspruch 9 oder 10, wobei Befreiung und Extraktion die physikalische Zerstörung der DNA/RNAenthaltenden Zellen umfasst, wobei die physikalische Zerstörung umfassen kann, dass das Reaktionsgefäß, das mindestens die DNA/RNA-adsorbierende Matrix, einen Lyse-/Extraktionspuffer und fragmentierte DNA umfasst, einem beliebigen aus Beschallung, Exposition gegenüber UV-Licht oder starker mechanischer Scherung unterzogen wird.

12. Verfahren nach Anspruch 11, wobei das Gefäß ferner Kügelchen umfasst, die bei der physikalischen Zerstörung der Zellen behilflich sind.

## Revendications

1. Procédé de prétraitement d'un échantillon d'une matrice d'adsorption d'ADN/ARN convenant pour l'extraction d'acide (désoxy)ribonucléique dérivé de cellules (« ADN/ARN cible ») de celui-ci, ledit procédé comprenant les étapes de :
a. isolement d'un échantillon d'une matrice d'adsorption d'ADN/ARN, ledit échantillon comprenant des cellules contenant l'ADN/ARN ; et
b. mise en contact de l'échantillon avec de l'ADN d'origine naturelle sous une forme fragmentée (« ADN fragmenté ») pour sensiblement bloquer lesdits sites de liaison d'adsorption d'ADN/ARN de ladite matrice d'adsorption, ledit ADN fragmenté n'étant « pas associé » à l'ADN/ARN cible desdites cellules contenant l'ADN/ARN,
dans lequel
1) l'ADN fragmenté ne fournit aucune réponse dans une expérience par PCR (réaction de polymérisation en chaîne) ciblant le gène de l'ARNr 16S avec 0,4 µM de chacune des amorces 341f selon SEQ ID NO : 4 et 518r selon SEQ ID NO : 5, en utilisant 10 ng de l'ADN fragmenté en tant que matrice, la réaction de PCR étant réalisée à 95°C pendant 5 minutes, suivi de 30 cycles de 95°C pendant 45 secondes, 55°C pendant 30 secondes, 72°C pendant 45 secondes et une étape finale de 72°C pendant 7 minutes, et^
2) l'ADN fragmenté a une longueur de fragment pouvant atteindre jusqu'à 300 paires de bases (pb) et/ou l'ADN fragmenté est dépuriné.

2. Procédé selon l'une quelconque des revendications précédentes, dans lequel l'ADN fragmenté est dépuriné.

3. Procédé selon la revendication 2, dans lequel la dépurination est réalisée par traitement à l'acide à un pH inférieur à 2,0.

4. Procédé selon l'une quelconque des revendications précédentes, dans lequel l'ADN d'origine naturelle est fragmenté par sonication et/ou par exposition à une lumière UV et/ou par cisaillement mécanique élevé et/ou par dépurination en combinaison avec un traitement thermique ou un traitement alcalin.

5. Procédé selon la revendication 3, dans lequel le traitement thermique a été réalisé à une température supérieure à 100°C et/ou dans lequel le traitement alcalin a été réalisé à un pH supérieur à 9.

6. Procédé selon l'une quelconque des revendications précédentes, dans lequel les cellules contenant l'ADN/ARN sont des cellules bactériennes, et l'ADN fragmenté provient de cellules eucaryotes, par exemple de cellules de vertébrés.

7. Procédé selon l'une quelconque des revendications précédentes, dans lequel l'ADN fragmenté est typiquement appliqué en une quantité de 0,5 à 50 mg par g de l'échantillon.

8. Procédé selon l'une quelconque des revendications précédentes, dans lequel l'échantillon d'une matrice d'adsorption d'ADN/ARN est un échantillon de sol.

9. Procédé d'extraction d'acide (désoxy)ribonucléique dérivé de cellules (« ADN/ARN cible ») d'une matrice d'adsorption d'ADN/ARN, ledit procédé comprenant les étapes de :
a. prétraitement d'un échantillon de matrice d'adsorption d'ADN/ARN selon le procédé selon l'une quelconque des revendications 1 à 8, dans lequel ledit échantillon comprend des cellules contenant l'ADN/ARN ;
b. libération de l'ADN/ARN cible desdites cellules contenant l'ADN/ARN et extraction dudit ADN/ARN cible dudit échantillon,
les étapes a et b étant facultativement réalisées en une étape.

10. Procédé selon la revendication 9, dans lequel l'ADN fragmenté utilisé pour le prétraitement est une partie d'un tampon d'extraction/de lyse utilisé pour la libération/l'extraction de l'ADN/ARN cible, ou dans lequel l'ADN fragmenté est présent dans une cuve de réaction où la libération et l'extraction de l'ADN/ARN sont réalisées par ajout d'un tampon d'extraction/de lyse et de la matrice d'adsorption d'ADN/ARN, dans lequel l'ADN fragmenté peut être présent dans la cuve sous une forme sèche, par exemple sous une forme lyophilisée.

11. Procédé selon la revendication 9 ou 10, dans lequel la libération et l'extraction entraînent la rupture physique des cellules contenant l'ADN/ARN, dans lequel la rupture physique peut entraîner la soumission d'une cuve de réaction comprenant au moins la matrice d'adsorption d'ADN/ARN, un tampon de lyse/d'extraction, et l'ADN fragmenté à l'un quelconque d'une sonication, d'une exposition à une lumière UV, et d'un cisaillement mécanique élevé.

12. Procédé selon la revendication 11, dans lequel la cuve comprend en outre des billes qui aident à la rupture physique des cellules.
